# EUROPEAN PATENT SPECIFICATION

(11) **EP 3 651 862 B1**
(45) Date of publication and mention of the grant of the patent: **17.02.2021**
(21) Application number: 18729092.9
(22) Date of filing: 31.05.2018
(51) Int. Cl.: A61Q 19/10, A61K 8/34, A61K 8/46, A61K 8/44

(54) **HIGH GLYCEROL COMPOSITION COMPRISING BLENDS OF ALKYL ISETHIONATE AND ALKYL TAURATE**
ZUSAMMENSETZUNG MIT HOHEM GLYCERINGEHALT MIT MISCHUNGEN VON ALKYLISETHIONAT UND ALKYLTAURAT
COMPOSITION À FORTE TENEUR EN GLYCÉROL CONTENANT DES MÉLANGES DE TAURATE D'ALKYLE ET D'ISÉTHIONATE D'ALKYLE

(30) Priority: 10.07.2017 EP 17180422
(43) Date of publication of application: 20.05.2020
(73) Proprietor: Unilever PLC, London, Greater London EC4Y 0DY (GB); Unilever N.V., 3013 AL Rotterdam (NL)
(72) Inventor: CARNALI, Joseph Oreste, Trumbull Connecticut 06611 (US); DAVE, Rajendra Mohanlal, Trumbull Connecticut 06611 (US)
(74) Representative: Fijnvandraat, Arnoldus
(86) International application number: PCT/EP2018/064336
(87) International publication number: WO 2019/011521

(56) References cited:
- EP-A2- 0 569 773
- WO-A2-2011/120780
- US-A1- 2009 062 406

## Description

### Field of the invention

The present invention relates to liquid cleanser compositions comprising specific blends of anionic surfactant and high glycerol (which provides excellent moisturization feel desirable to consumer). Preferably, the blend of anionics is further blended with amphoteric surfactants, but preferably the amphoteric is minimized relative to total surfactant so that deposition of benefit agent can be maximized. Preferably, compositions are clear. Preferably, they are isotropic. Preferably, they are clear and isotropic.

### Background of the invention

It is desirable to formulate compositions which have relatively mild surfactants. One combination of surfactants which is particularly desirable is combination of alkyl isethionate (e.g., fatty acid esters of isethionate acid such as sodium lauroyl or sodium cocoyl isethionate) and alkyl taurate (e.g., alkyl taurate amides such as N-methyl taurate). Such surfactant mixtures have the advantage that they are sulfate-free. Further, they offer the ability to formulate isotropic systems at neutral and slightly acidic pH. Thus they allow the use of more skin friendly preservation systems. It is also desirable to provide compositions which have high levels of glycerol (e.g., 20% or more, preferably 25% to 60%, preferably 30 to 55% by wt.) because the glycerol provides excellent sensory feel (e.g., moisturization feel).

A drawback of such compositions containing alkyl isethionates, alkyl taurates, and glycerol is that the combination is poorly soluble, so that it is not possible to obtain useful levels of the mild surfactants (e.g., greater than or equal to 5 % or 6% by wt.) when greater than or equal to 20% by wt. glycerol is used while still maintaining a clear, isotropic formulation.

A partial solution to this insolubility problem when formulating cleansing compositions is to incorporate some level of amphoteric surfactant because amphoteric surfactants are mild in their own right and help to improve the solubility of alkyl isethionate/ alkyl taurate blends.

They also aid in increasing the viscosity of the formulation, making it easier to meet consumer expectations without the addition of thickeners.

Even further, it is preferred, and desired that compositions of alkyl isethionates, alkyl taurates, glycerol, and amphoteric surfactants be clear, and isotropic, preferably both, where isotropic is defined by an Nephelometric Turbidity Unit ("NTU") value of 20 or below, preferably 10 or below.

At the high levels of glycerol desired in the subject invention, it has been noted that the level of amphoteric required to have compositions remain clear and isotropic also rises. Thus to be compatible with high level of glycerol (20% or more, preferably 25% or more), it can typically require use of 30% or more amphoteric. However, while use of amphoteric surfactant (e.g., betaine) is desired, as noted above, it is also desired that the level not be too high as a percent of all surfactant because this is believed to affect deposition of other benefit agents found in the compositions. Such benefit agents can include emollients, hydrocarbon and/or silicone oils, nutrients, antimicrobial agents, and particulates. In isethionate/taurate systems of our invention, there is thus a need to find a way to incorporate maximum glycerol at minimum amount of amphoteric while maintaining a clear, isotropic liquid.

Unexpectedly, applicants have found critical ratios of isethionate to taurate which in fact allows maximum incorporation of glycerol while minimizing level of amphoteric or preferably both, while maintaining clear, isotropic liquids.

U.S. Publication No. 2009/0062406 to Loeffler discloses compositions comprising mixtures of sodium cocoyl isethionate, sodium methyl taurate and alkyl betaine which yield clear, isotropic, low viscosity solutions characterized by NTU of 10 or below. Compositions taught are 20% cocobetaine, 5% isethionate and 5% taurate (i.e., betaine is 66% of total surfactant and there is 1:1 ratio of isethionate to taurate); and 10% betaine, 10% isethionate, 10% taurate (i.e., betaine is 33% of total surfactant and there is a 1:1 ratio of isethionate to taurate). For clarity of representation, the weight ratio of isethionate to taurate will be stated below as a decimal value R. For example, 1:1 weight ratios of isethionate to taurate correspond to R = 1.0.

WO2011120780 A2 discloses a personal cleanser comprising 1 to 30% of a surfactant system comprising fatty acyl isethionate and an alkanoyl surfactant.

US2010075881 A1 discloses stable liquid cleansing compositions comprising fatty acyl isethionate and high levels of glycerine.

In Example 6 below, applicants prepared a series of compositions having 10% total isethionate and taurate at the following ratios of isethionate to taurate: 7.5/2.5, 5.0/5.0, 2.5/7.5, 0/10 (R = 3, 1, 0.333 and 0 respectively). Applicants added cocamidopropyl betaine (CAPB) at levels ranging from 4.76% (0.5/10.5) to 33% (5.0/15) of total surfactant; and measured transparency after two months. Table 7 demonstrates that, at a fixed isethionate/taurate ratio, decreased CAPB leads to increased turbidity (higher NTU values). The table also shows a particularly efficient reduction in turbidity as the ratio of taurate to isethionate is higher (or conversely ratio of isethionate to taurate is lower). This is also true when there is only taurate present (ratio of 0/10). As indicated, the Loeffler reference discloses ratios of taurate to isethionate of 1:1. If one of ordinary skill in the art chose to increase the ratio of taurate to isethionate (and there is no particular incentive to do so), they might find less CAPB is needed to achieve decent (i.e., low) level of turbidity (as we show in right two columns of Table 1 versus left two columns). However, the reference does not relate to utilizing high levels of glycerol and fails to understand the relationship between taurate to isethionate levels, glycerol and turbidity.

Specifically, the person of ordinary skill, as seen from Table 7, might be driven to eliminate isethionate altogether and use only taurate (0 to 10 ratio of isethionate to taurate as seen in far right column). As we clearly and unexpectedly demonstrate, however (see Table 1), when dealing with glycerol, and trying to minimize level of CAPB in a glycerol rich system, there is a critical window. When there is too much taurate (e.g. at 0% isethionate), 40% CAPB is required to yield isotropic solution when 30% glycerol is used (far right column). By contrast, we can obtain an isotropic solution when the same 30% glycerol is used and we use only 30% CAPB; the key is to use a ratio of 1/6 isethionte to taurate. At a ratio of 1/5 we can obtain an isotropic solution using even higher 35% glycerol while still using only 30% CAPB. This critical window where amphoteric is minimized in high glycerol systems based on ratio of surfactants is not recognized in Loeffler because formation of isotropic, glycerol rich compositions at minimal CAPB is simply not contemplated.

### Summary of the Invention

Quite unexpectedly, applicants have discovered a specific window, defining a particular ratio of isethionate to taurate, in combination with specific ratio of the combined anionic surfactants to amphoteric (maximizing level of non-sulfated anionic surfactant) wherein high levels of glycerol can be used. Further, even though some level of amphoteric is needed, by defining specific ratios of one anionic to the other, high glycerol systems are made, the amount of amphoteric surfactant used is minimized (which helps deposition); and clear, isotropic compositions are made.

More specifically, the invention relates to cleansing compositions comprising:
1) 5 to 20%, preferably 6 to 15% by wt. of a surfactant system comprising:
   a) alkali metal acyl isethionate;
   b) alkyl taurate; and
   c) amphoteric, preferably, alkyl betaine,
   wherein weight ratio of (a) to (b) Is 1:1.5 to 1:6 (R=0.667 to 0.166), preferably 1:1.5 to 1:5 (R=0.667 to 0.2); more preferably 1:19 to 1:5 (R=0.53 to 0.2);
   wherein ratio of ((a) plus (b))/(c) is 1:1 to 9:1; and
2) 20 to 60%, preferably 25 to 55% by wt. glycerol.

### Detailed description of the invention

Except in the examples, or where otherwise explicitly indicated, all numbers in this description indicating amounts of material or conditions of reaction, physical properties of materials and/or use are to be understood as modified by the word "about."

As used throughout, ranges are used as shorthand for describing each and every value that is within the range. Any value within the range can be selected as terminus of the range. The use of and/or indicates that any one from the list can be chosen individually, or any combination from the list can be chosen.

For the avoidance of doubt, the word "comprising" is intended to mean "including" but not necessarily "consisting of" or "composed of." In other words, the listed steps or options need not be exhaustive.

Unless indicated otherwise, all percentages for amount or amounts of ingredients used are to be understood to be percentages by weight based on the active weight of the material in the total weight of the composition, which total is 100%.

### Surfactant System

The invention relates to compositions comprising 5 to 20%, preferably 6 to 15% by weight of a surfactant system which comprises (a) alkali metal acyl isethionate, (b) alkali metal alkyl taurate and (c) an amphoteric surfactant, preferably an alkyl betaine.

Using specific ratio of surfactants, applicants can provide high glycerol systems (greater than or equal to 20%), minimize amphoteric (some benefit for harshness and viscosity, but minimal effect on reduction of deposition, i.e., desirable higher levels of deposition are maintained), all while maintaining clear, isotropic compositions. It was quite surprising that one could minimize amphoteric (using high glycerol systems) while maintaining clear compositions when critical ratios of isethionate to taurate are used. Indeed, when only taurate is used, high levels of amphoteric (CAPB) are needed but, using only slightly less taurate (high ratios of taurate to isethionate disclosed in our invention), much less amphoteric can be used while still obtaining isotropic liquid.

The first component of the surfactant systems of the Invention is alkali metal acyl isethionate.

Acyl isethionate surfactant is commonly produced by the direct esterification of a fatty acid (e.g., C₁₀ to C₁₆ fatty acid such as lauric acid) and isethionate (e.g., HOCH₂CH₂SO₄M^{+,} where M⁺ may be, for example, a sodium or potassium counterion) in a process commonly known as the "DEFI" process, where DEFI refers to directly esterified fatty acid isethionate.

Preferred isethionate sulfonates include cocoyl isethionate and lauroyl isethionate, preferably having sodium or potassium as counterions.

A second component of surfactant system of our Invention are alkyl taurates, e.g., alkyl taurate amides. Such alkyl taurate amides may be made by reaction of a taurine; methyl taurine; or a corresponding taurate salt (e.g., NH₂CH₂CH₂SO₃ M⁺, where M⁺ may be sodium or potassium counterion) with the appropriate fatty acid.

Preferably alkyl taurate amides include sodium methyl cocoyl taurate and sodium methyl lauroyl taurate.

The third required component of surfactant systems of the invention is amphoteric surfactant. Suitable amphoteric surfactants include derivatives of acyl ethylenediamines. A preferred example of compounds in this class is sodium lauroamphoacetate. Suitable amphoteric surfactants also include derivatives from the alkyl betaine class, including alkyl betaines, alkyl amidopropyl betaines, and alkyl amidopropyl hydroxysultaines. Preferred examples of compound in this class are coco betaine and cocamidopropyl betaine - commonly referred to as CAPB.

As indicated, amphoteric surfactants are typically used as partial replacement for harsher anionic surfactants. However, it is preferred to minimize amount of amphoteric in order to aid deposition.

Selection of specific ratios of isethionate to taurate unexpectedly has been found to provide a window where use of high levels of glycerol is permitted while minimizing amount of amphoteric surfactant used, all while maintaining clear isotropic solutions.

Specific window of isethionate to taurate is 1:1.5 to 1:6 (R=0.66 to 0.166), preferably 1:1.9 to 1:5. Thus, preferred ratios R are taurate-rich but contain finite, non-zero levels of isethionate.

Further, preferred ratio of isethionate plus taurate to amphoteric is 1:1 to 9:1. The total surfactant blend is always majority anionic.

A further component of the invention is glycerol. As indicated, the key is to maximize amount of glycerol (20% or more) while minimizing amount of amphoteric, all while retaining clear, isotropic compositions.

### Skin or Hair Benefit Agents

In the same composition of the invention, 0 to 30% by wt., preferably 0.1 to 10%, more preferably 0.1 to 5% by wt. skin or hair benefit agent is used. Preferably, the benefit agent is an oil soluble emollient or moisturizing oil. These are molecules which increase hydration by various mechanisms which may include prevention of water loss (occlusive agents), attracting moisture (humectants); or which restore natural moisturizing factors to the skin (e.g., amino-lipids). Preferred moisturizers include petrolatum and silicone. Preferably, moisturizer is a vegetable or triglyceride oil. Preferred oils include sunflower seed oil and soybean oil. The moisturizer may be an ester of long chain [C₁₄-C₃₀] fatty acid, such as isopropyl palmitate.

Some naturally restorative agents and moisturizers include:
a) vitamins such as vitamin A and E, and vitamin alkyl esters such as vitamin C alkyl esters;
b) lipids such as cholesterol, cholesterol esters, lanolin, sucrose esters, and pseudo-ceramides;
c) liposome forming materials such as phospholipids, and suitable amphophilic molecules having two long hydrocarbon chains;
d) essential fatty acids, poly unsaturated fatty acids, and sources of these materials;
e) triglycerides of unsaturated fatty acids such as sunflower oil, primrose oil, avocado oil, almond oil;
f) vegetable butters formed from mixtures of saturated and unsaturated fatty acids such as shea butter;
g) minerals such as sources of zinc, magnesium and iron; and
h) silicone oils, gums, modifications thereof such as linear and cyclic polydimethylsiloxanes, amino, alkyl and alkyl aryl silicone oil.

Water soluble benefit agents may also be used. Preferred water soluble agents include glycerol, sorbitol, polyalkylene glycols and mixtures thereof.

If used, depending on amount and miscibility of benefit agent in the isotropic surfactant chassis, the chassis may still maintain clarity. However, even if benefit agent renders chassis anisotropic, the sulfate-free and neutral-to-slightly-acidic pH benefits discussed above are still retained. Also, the benefit of excellent sensory feel and good deposition will still be present.

Although compositions of the invention do not require external structurants, when oil soluble benefits as noted above are used, it is preferably to use structurants.

### Structurant

Preferably, compositions of the invention comprise 0.1 to 10% by wt., preferably 0.5 to 7% by wt. of a structurant. The structurant may be a water soluble or water dispersible polymer which can be a cationic, anionic, amphoteric or nonionic polymer for enhancing viscosity.

Examples of water soluble/or dispersible polymers useful in the present invention include the carbohydrate gums such as cellulose gum, microcrystalline cellulose, cellulose gel, hydroxyethyl cellulose, hydroxypropyl cellulose, sodium carboxymethylcellulose, hydroxymethyl or carboxymethyl cellulose, methyl cellulose, ethyl cellulose, guar gum, gum karaya, gum traganth, gum Arabic, gum acavia, gum agar, xanthan gum and mixture thereof; modified and non-modified starch granules with gelatinization temperature between 30 to 85°C, and pregelatinized cold water soluble starch; polyacrylate; Carbopols; alkaline soluble emulsions polymer such as Aculyn 28, Aculyn 22 or Carbopol Aqua SF1; cationic polymers such as modified polysaccharides including cationic guar available from Rhone Poulenc under the trade name Jaguar C13S, Jaguar C14S, Jaguar C17, or Jaguar C16, BF Guar C17 from Lamberti, Aqua D4091 or Aqua D4051 from Aqualon; cationic modified cellulose such as UCARE Polymer JR30 or JR 40 from Amerchol; N-Hance 3000, N-Hance 3196, N-Hance CPX215 or N-Hance GPX 196 from Hercules; synthetic cationic polymer such as Merquat 100, Merquat 280, Merquat 281 and Merquat 550 by Nalco; cationic starches, e.g., StaLok® 100, 200, 300 and 400 made by Staley Inc.; cationic galactamannans based on guar gum of Galactasol 800 series by Henkel, Inc.; Quadrisect Um-200, and Polyquaternium-24.

Gel forming polymers such as modified or non-modified starch granules, xanthan gum, Carbopol, alkaline-soluble emulsion polymers and cationic guar gum such as Lamberti BF Guar C17, and cationic modified cellulose such as UCARE Polymer JR 30® or JR 40® are particularly preferred for this invention.

A preferred structuring copolymer is the polymerization product (e.g., additive polymerization product) of (1) a first ethylenically unsaturated monomer; (2) a second ethylenically unsaturated monomer; (3) (meth)acrylate monomer and (4) associative monomer (generally random in structure; preferably copolymers are linear).

The first monomer of (1) may be di-acid of formula:

HOOC-CR¹=CR²-COOH (I),

a cyclic anhydride precursor of diacid (I), the anhydride having the formula: 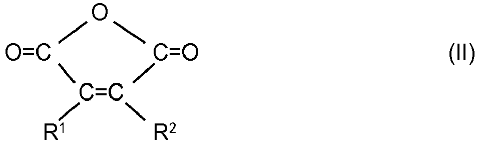 and combinations thereof,
wherein R¹ and R² are individually selected from H, C₁-C₃ alkyl, phenyl, chlorine and bromine and, in one or more embodiments, are preferably individually selected from H and C₁-C₃ alkyl.

Preferred monomers include maleic acid and maleic acid anhydride. It may comprise 0 to 10%, preferably 0.1 to 5% by wt. on total wt. of monomer charge.

The second monomer (2) can be acrylic acid, methacrylic acid and combinations thereof. It can be used at 15-60% by wt. based on total monomer charges.

The third (meth)acrylate monomer can be C₁ to C₈ alkyl esters of acrylic acid, C₁ to C₈ alkyl esters of methacrylic acid and combinations and can be 30-75% by wt. based on total monomer charge.
a) The associative monomer has the formula:

   R⁴-CH=C(R³)-C(O)-O-(R⁵O)ₐ-R⁶ (III)

   wherein:
   R³ and R⁴ are independently selected from H and C₁₋₃ alkyl,
   each R⁵O is independently an oxyalkylene unit having from 2 to 4, preferably from 2 to 3 carbon atoms,
   R⁶ is selected from:
      linear and branched alkyl having from 8 to 40, preferably from 8 to 30, more preferably from 10 to 22 carbon atoms, and
      alkaryl, the alkyl group of which has from 8 to 40, preferably from 8 to 30, more preferably from 10 to 22 carbon atoms, such alkyl group being linear or branched, said alkaryl preferably being alkylphenyl; and
   a has a value of from 6 to 40, preferably from 15 to 35, most preferably from 20 to 30.

Of particular interest in one or more embodiments is an associative monomer of the formula:

CH₃[CH₂]_{b}-CH₂-[OCH₂CH₂]ₐ -O-C(O)C(R³)=CH(R⁴) (IV)

in which R³, R⁴ and a are as described above, and b has a value of from 6 to 38, preferably from 6 to 28, and more preferably from 8 to 20.

In the Formula III and Formula IV monomers, R³ is preferably a methyl group and R⁴ is preferably H. In the above described associative monomers, a and b represent the number of their respective oxyalkylene and -CH₂- repeat units and generally are integers. In one or more embodiments of interest a is greater than or equal to b.

The associative monomer may be employed in amounts of from 1 to about 25 wt. %, preferably from 2 to 20 wt. %, and more preferably from 2 to 15 wt. %, based on total monomer added. In one or more embodiments of particular interest the amount of associative monomer employed is from 5 to 12 wt. %, based on total monomer added.

Some compositions may contain water-soluble polymers in amounts of 0.005 to 5% by wt.

Examples of water soluble polymers include high molecular weight polyethylene glycols such as Polyox® WSR-205 (PEG 14M), Polyox® WSR-N-60K (PEG 45M), and Polyox® WSR-301 (PEG 90M); the carbohydrate gums such as cellulose gum. Hydroxyethyl cellulose, hydroxypropyl cellulose, sodium carboxymethylcellulose, methyl cellulose, ethyl cellulose, guar gum, gum karaya, gum tragacanth, gum arabic, gum acacia, gum agar, and xanthan gum; modified starch granules and pregelatinized cold water soluble starch; cationic polymer such as modified polysaccharides including cationic guar available from Rhodia under the trade name Jaguar®; cationic modified cellulose such as UCARE Polymer JR 30 or JR 40 from Amerchol; N-Hance® 3000, N-Hance® 3196, N-Hance® GPX 215 or N-Hance® GPX 196 from Hercules; synthetic cationic polymers such as Merquat® 100, Merquat® 280, Merquat® 281 and Merquat® 550 sold by Nalco. Additional molecules include glycerol and sorbitol.

The water soluble polymers may be used individually or as combinations of two or more polymers from the same or different classes. High molecular weight polyethylene glycols Polyox® WSR-301 (PEG 90M) and Polyox® WSR-N-60K (PEG 45M) and guar derivatives such as Jaguar® S, Jaguar® C17, and Jaguar® C13, and synthetic cationic polymers such as Merquat® 100 are particularly desired.

### Preservatives

Personal product formulations provide good media for growth of microbes. Microbial action can be manifested in terms of hydrolysis, oxidation or reduction and may cause off-odors, changes in color, adverse change in pH, breaking of emulsions, and changes in product texture. Thus good preservation systems are required to prevent microbial growth, spoiling of product, and infection of skin and hair. The preservative should be effective against Gram-negative and Gram-positive bacteria as well as fungi (molds and yeasts).

An effective preservative is a chemical agent which will prevent microbial growth in the product, making it safe and increasing shelf life.

Optimal preservation system should provide broad spectrum activity and be effective over the shelf-life of the product. As microorganisms multiply in the aqueous phase of formulations, the preservation system should also be water-soluble. Where formulations contain appreciable levels of oils, the system should favor partitioning into the aqueous phase. Ideally, the preservation system should be effective over wide pH range, colorless and safe in use. It should be non-irritating, non-sensitizing and preferably non-poisonous. Ideally, while eliminating pathogenic organisms in the formulation while in storage, it should leave symbiotic organisms on the skin in peace after application of the formulation to the skin, hair or mucous membrane.

Some preferred preservatives include:
1) Parabens, for example, methyl-, ethyl-, propyl-, iso-butyl-, and butyl-paraben;
2) Formaldehyde-releasing preservatives, for example, formaldehyde, quaternium-15, dimethyl-dimethyl (DMDM) hydantoin, imidazolidinyl urea, diazolidinyl urea, sodium hydroxymethylglycinate, and 2-bromo-2-nitropropane-1,3-diol;
3) Isothiazolones, such as chloromethyl-isothiozolinone (CMIT), methyl-isothiazolinone (MIT) or benz-isothiazolinone (BIT);
4) Halogen-organic actives, such as idopropynyl butylcarbamate and methyl-dibromo glutaranitrile;
5) Organic acids such as benzoic acid, dehydroacetic acid, salicylic acid, lactic acid and sorbic acids;
6) Other, including chloroacetamide, phenyloxyethanol and triclosan.

Additional suitable preservatives for personal care products can be found in "Preservatives for Cosmetics Manual, 2nd edition", by David S. Steinbens, 2006 and in "Preservatives for Cosmetics", D.C. Steinberg, Allured Publishing Corp., ISBN #0-93170-54-5. Such agents are typically employed at 0.1-1%, more preferably at 0.5-0.7% of the personal product formulation.

The organic acids noted are particularly preferred. Especially preferred are organic acids having pKa between about 4.0 and 5.5, preferably 4.0 and 5.0.

No preservative is ideal for all situations. For example, parabens are relatively non-irritant, but partition in favor of oil phase and are inactivated by some surfactants. Formaldehyde-retaining preservatives have broad effectiveness spectrum, but are irritant and banned in some countries.

As indicated, benzoic acid is a preferred preservative.

Optionally, the compositions of this invention may further comprise one or more additional ingredients. Non-limiting examples of such additional ingredients are, for example, colorants, pigments, opacifiers, fragrance (whether encapsulated or present as free-fragrance), emotive oils, vitamins and vitamin derivatives, abrasives, optical agents (including for example, reflective particles and interference pigments), pH adjusters, plant extracts, essential oils, preservatives, antioxidants, antimicrobials, viscosity modifiers, humectants, beard wetting agents, sensory agents, fatty acid soap, and skin and/or hair benefit agents (e.g., aloe, allantoin, panthenol, alpha-hydroxy acids, phospholipids, botanical oils, and amino acids to name a few). The selection and amount of any individual additional ingredient depends upon factors that include the particular ingredient, the properties desired, and the intended use of the composition in which it is employed. For example, fragrance is typically employed in an amount of 0.1 to 3.0% by weight of the composition, or higher. For many compositions, the total amount of such additional ingredients is 0.01 to 30% by weight, more particularly, 0.1 to 15% by weight, even more particularly, 1 to 10% by weight, based on the total weight of the composition. In one or more embodiments, the total amount of such additional optional ingredients is 0.5 to 5% by weight.

Compositions are aqueous based and comprise typically 30-90% by wt. water. Water is balance after all ingredients noted above are accounted for.

### pH

In general, pH of compositions of the invention is in the range of 4.5 to 7.5. This permits use of a wide variety of preservation systems.

### Protocol

### Transparency of the compositions

The clarity of the compositions is quantified by measuring their turbidity - defined as the degree to which light is scattered by inhomogeneities in the composition. The measured turbidity depnds on the wavelength of light used in the measurement and on the angle at which the detector is positioned. The Nephelometric Turbidity Unit (NTU) is measured using white light scattered at a 90° angle from the incident beam according to US EPA Method 180.1, "Turbidity". The white light source typically has a peak spectral output in the range of 400-680 nm. This information has been obtained from the website https://or.water.usgs.gov/grapher/fnu.html. Turbidity measurements were made using a Hach 2100N Turbidimeter calibrated according to the manufacturer's instructions against standards of known NTU value. Higher NTU value corresponds to higher turbidity and lower transparency.

### Examples

The following examples are chosen to demonstrate the following:
Example 1 establishes the optimal ratio of isethionate to taurate for accomadation of glycerol into a clear, isotropic solution using the least amount of alkyl betaine. At 9% total surfactant, this ratio is found to lie in the range R = 0.667 to 0.166.
Example 2 shows that this optimal ratio applies as well to higher and lower total surfactant levels, as might be found in a wide range of personal wash products.
Example 3 demonstrates that the invention is valid for amphoteric surfactants beyond alkyl betaines, and alkyl taurates beyond cocoyl taurate.
Example 4 establishes that the pH range of compositions of the invention is about pH 4.5 to 7.5. This permits use of variety of preservation systems.
Example 5 shows various compositions (A to G) each demonstrating different aspects of the invention.
Example 6 shows how a person skilled in the art would not have readily recognized criticalities involved in ratios of isethionate to taurate when seeking to minimize amphoteric and maintain isotropic liquids when glycerol, particularly high levels of glycerol, is used in the composition. Rather, they would be led to believe that 100% taurate systems are fine when, in fact, at least certain levels of isethionate are needed to minimize levels of amphoteric while using much higher levels of glycerol and maintaining transparency. Specifically, to optimize the solubility of an isethionate/taurate blend with the least required amount of added alkyl betaine (in desired high glycerol systems) requires the critical isethionate/taurate ratios claimed in the present patent application.

### Example 1. Maximum allowable Glycerol level at 9% total surfactant.

The purpose of this example was to identify the ratio(s) of isethionate/taurate which allow(s) for the maximal incorporation of glycerol using the minimum level of amphoteric, e.g., cocamidepropyl betaine ("CAPB"), all while maintaining a clear, isotropic solution. Use of minimal CAPB levels is preferred for body wash and shampoo formulations as it is believed that high CAPB levels disfavor deposition of benefiting agents. For this example, a series of varying isethionate/taurate weight ratios, 4/1, 2/1, 1/1, 1/1.5, ½, 1/3, ¼, 1/5, 1/6, and 0 (R = 4, 2, 1, 0.666, 0.5, 0.333, 0.25, 0.2, 0.166, and 0, respectively) were prepared. At each ratio, the cocamidopropyl betaine (CAPB) level was varied in such a way that the CAPB made up 10 to 50% of the total surfactant level, which was maintained at 9% in this example. For each surfactant composition, the maximum glycerol content still yielding a clear, isotropic solution was determined by constructing individual compositions, adjusting the pH to 7.0, heating to 65°C with stirring and then equilibrating at 23°C for two months, by which time no further changes in physical appearance were noted. After equilibration, the transparency of the individual mixtures was measured as described in the protocol. The highest glycerol content (wt.%) which still resulted in a clear, isotropic solution is reported in Table 1.

**Table 1. Maximal glycerol level (wt. %) still yielding a clear, isotropic solution at 9% total surfactant blend**

| | *IsethionatelTaurate ratio (wt.%*/*wt.%)* | | | | | | | | | |
|---|---|---|---|---|---|---|---|---|---|---|
| | 4/1 | 2/1 | 1/1 | 1/1.5 | 1/2 | 1/3 | 1/4 | 1/5 | 1/6 | 0 |
| *R* | 4.0 | 2.0 | 1.0 | 0.666 | 0.5 | 0.333 | 0.25 | 0.2 | 0.166 | 0.0 |

| *CAPB* %* | *Glycerol level, %* | | | | | | | | | |
|---|---|---|---|---|---|---|---|---|---|---|
| 50 | 35 | 40 | 60 | 60 | 55 | 55 | 50 | 50 | 45 | 45 |
| 40 | 20 | 30 | 50 | 50 | 50 | 45 | 40 | 40 | 40 | 30 |
| 30 | <10 | <10 | 30 | 40 | 40 | 37.5 | 35 | 35 | 30 | 25 |
| 20 | <10 | <10 | <10 | 20 | 35 | 32.5 | 30 | 20 | 20 | 20 |
| 10 | <10 | <10 | <10 | <10 | 25 | 25 | 10 | <10 | <10 | <10 |
| 5 | <10 | <10 | <10 | <10 | <10 | 10 | <10 | <10 | <10 | <10 |

| | | | | | | | | | | |
|---|---|---|---|---|---|---|---|---|---|---|
| *CAPB % is of total surfactant | | | | | | | | | | |

Table 1 illustrates that isethionate/taurate ratios of 1/1 (R = 1) and higher require 30% or more of CAPB in order to be compatible with 20% glycerol. Mixtures richer in taurate are more glycerol tolerant at lower CAPB levels. There is an optimal isethionate/taurate ratio range, from about 1/1.5 to about 1/5 (R = 0.666 to 0.2) in which the system is especially tolerant to glycerol at low CAPB levels. For example, 30% or more glycerol can be accommodated by 20% or less CAPB. In the limit of zero isethionate (R = 0), glycerol tolerance is seen to be reduced. This is particularly counter-intuitive.

### Example 2. Maximum allowable Glycerol level at 6-12% total surfactant.

The purpose of this example was to identify the ratio(s) of isethionate/taurate which allow(s) for the maximal incorporation of glycerol using the minimum level of amphoteric ("CAPB"), all while maintaining a clear, isotropic solution. Use of minimal CAPB levels is preferred for body wash and shampoo formulations as it is believed that high CAPB levels disfavor deposition of benefiting agents. For this example, the total surfactant level was kept constant at 6%, 9%, and 12%, while the isethionate/taurate weight ratio R was varied in increments from 0 to 3. At each ratio, the cocamidopropyl betaine (CAPB) level was varied in such a way that the CAPB made up 25% or 33% of the total surfactant level. For each surfactant composition, the maximum glycerol content still yielding a clear, isotropic solution was determined by constructing individual compositions, adjusting the pH to 7.0, heating to 65°C with stirring and then equilibrating at 23°C for two months, by which time no further changes in physical appearance were noted. After equilibration, the transparency of the individual mixtures was measured as described in the protocol. The highest glycerol content (wt.%) which still resulted in a clear, isotropic solution is reported in Table 2 for 25% CAPB and in Table 3 for 33% CAPB.

**Table 2. Maximal glycerol level (wt. %) still yielding a clear, isotropic solution at fixed total surfactant blend levels of 6, 9, and 12% and with CAPB as 25% of the total surfactant.**

| *Isethionate*/ *Taurate (wt*/*wt)* | *6% total surfactant* | *9% total surfactant* | *12% total surfactant* |
|---|---|---|---|
| R | *Glycerol level, %* | | |
| 0.0 | 30 | 15 | 10 |
| 0.286 | 37.5 | 30 | 22.5 |
| 0.5 | 42.5 | 35 | 32.5 |
| 0.8 | 32.5 | 30 | 22.5 |
| 1.25 | 17.5 | 10 | <5 |
| 2.0 | <5 | 10 | <5 |
| 3.5 | <5 | <5 | <5 |

**Table 3. Maximal glycerol level (wt. %) still yielding a clear, isotropic solution at fixed total surfactant blend levels of 6, 9, and 12% and with CAPB as 33% of the total surfactant.**

| *Isethionate*/ *Taurate (wt*/*wt)* | *6% total surfactant* | *9% total surfactant* | *12% total surfactant* |
|---|---|---|---|
| R | *Glycerol level, %* | | |
| 0.0 | 32.5 | 25 | 17.5 |
| 0.1428 | 37.5 | 35 | 32.5 |
| 0.333 | 42.5 | 35 | 32.5 |
| 0.6 | 55 | 40 | 37.5 |
| 1 | 32.5 | 30 | 27.5 |
| 1.666 | 10 | 10 | 9 |
| 3.0 | <5 | <5 | <5 |

Tables 2 and 3 illustrate that the trend observed at 9% total surfactant level carries over to higher (12%) and lower (6%) surfactant levels. With 33% of the total surfactant as CAPB, the maximum glycerol level which can be accommodated as a clear, isotropic solution is maximal (35% up to 55%) for an isethionate/taurate ratio in the range of 0.14 to 0.6. These ratios correspond approximately to the claimed ratio range of 1/1.5 to 1/6 (R = 0.666 to 0.166).

At 25% total surfactant as CAPB, the maximum glycerol level (about 35%) is accommodated at an isethionate/taurate ratio of 1/2, corresponding to R = 0.5.

Again, isethionate/taurate ratios higher than 1/1 (R = 1) require more than 33% or 25% of CAPB in order to be compatible with 20% glycerol and mixtures richer in taurate are more glycerol tolerant at lower CAPB levels. However, in the limit of zero isethionate, the glycerol solubilizing capacity is reduced at all total surfactant levels.

### Example 3

In this example, the scope of the invention is expanded to include other types of alkyl betaines beyond CAPB, other types of amphoteric surfactants beyond alkyl betaines, and other types of sodium methyl alkyl taurates beyond sodium methyl cocoyl taurate (SMCT). Identification is again made of the ratio(s) of isethionate/taurate which allow(s) for the maximal incorporation of glycerol for a minimal level of amphoteric surfactant, all while maintaining a clear, isotropic solution. In this example, cocamidopropyl betaine (CAPB), coco betaine, or lauroamphoacetate are used as the amphoteric and sodium methyl cocoyl taurate (SCMT) or sodium methyl lauroyl taurate (SMLT) is used as the alkyl taurate. Use of minimal amphoteric surfactant levels is preferred for body wash and shampoo formulations as it is believed that high amphoteric levels disfavor deposition of benefiting agents. For this example, a series of varying isethionate/taurate weight ratios, 2/1, 1/1, ½, 1/3, 1/5, 1/11, and 0 (R = 2, 1, 0.5, 0.333, 0.2, 0.09, and 0, respectively) were prepared. At each ratio, the amphoteric surfactant level was held at a 3% level, 33% of the total surfactant level - which is kept constant at 9%. Thus, the combination of isethionate and taurate makes up a fixed 6% of the entire composition. For each surfactant composition, the maximum glycerol content still yielding a clear, isotropic solution was determined by constructing individual compositions, adjusting the pH to 7.0, heating to 65°C with stirring and then equilibrating at 23°C for two months, by which time no further changes in physical appearance were noted. After equilibration, the transparency of the individual mixtures was measured. The highest glycerol content (wt.%) which still resulted in a clear, isotropic solution is reported in Table 4.

**Table 4. Maximal glycerol level (wt. %) still yielding a clear, isotropic solution at fixed total surfactant blend levels of 9% with the amphoteric surfactant level fixed at 3%.**

| *Isethionate*/ *Taurate (wt*/*wt)* | *A. Coco betaine as amphoteric SMCT as alkyl taurate* | *B. Amphoacetate as amphoteric SMCT as alkyl taurate* | *C. CAPB as amphoteric SML T as taurate* |
|---|---|---|---|
| *R* | *Glycerol level,* % | | |
| 0.0 | 22.5 | 15 | 40 |
| 0.09 | 25 | | 42.5 |
| 0.2 | 28 | 27.5 | 48 |
| 0.33 | 15 | | 48 |
| 0.5 | 15 | 37.5 | 30 |
| 1.0 | 15 | 20 | 10 |
| 2.0 | 2 | 2 | 2 |

Table 4 illustrates that the trend observed in Example 1 for CAPB as the amphoteric surfactant and sodium methyl cocoyl taurate (SMCT) as the taurate extends to other amphoteric surfactants and other alkyl taurates. In column A, coco betaine is used as the amphoteric surfactant, in column B lauryl amphoacetate is used as the amphoteric surfactant, and in column C sodium methyl lauroyl taurate (SMLT) is used as the alkyl taurate. With 33% of the total surfactant as amphoteric surfactant, the maximum glycerol level which can be accommodated as a clear, isotropic solution is maximal for an isethionate/taurate ratio in the range of 0.09 to 0.5. These ratios correspond approximately to the claimed ratio range of 1/1.5 to 1/6 (R = 0.666 to 0.166). Again, isethionate/taurate ratios higher than 1/1 (R = 1) require more than 33% of amphoteric in order to be compatible with 20% glycerol and mixtures richer in taurate are more glycerol tolerant at lower amphoacetate levels. However, in the limit of zero isethionate (R = 0), the glycerol solubilizing capacity is reduced.

### Example 4

In this example, the pH range of the invention is established. A specific ratio of isethionate-to-taurate of ½ (R=0.5) is chosen, though other ratios within the preferred range R = 0.666 to 0.166 also apply. Similarly, cocamidopropyl betaine (CAPB) is used as the amphoteric surfactant and sodium methyl cocoyl taurate (SMCT) is chosen as the alkyl taurate, though other choices for the amphoteric and/or the alkyl taurate show the same result. The total surfactant level is held constant at 9% and the level of CAPB is kept at 1.8%, or 20% of the total surfactant, because it is believed that high amphoteric levels disfavor deposition of benefiting agents. Thus, the combination of isethionate and taurate makes up a fixed 7.2% of the entire composition. For each surfactant composition, the glycerol content was set to 30% of the total composition. Preparation consisted of heating to 65°C with stirring, adjusting the pH as indicated below, and then equilibrating at 23°C for two months, by which time no further changes in physical appearance were noted. After equilibration, the transparency of the individual mixtures was measured. The specific compositions tested are reported in Table 5.

**Table 5. Compositions with a constant 9% level of total surfactant, with 20% of the surfactant as amphoteric CAPB, with an isethionate/taurate ratio of 1:2, and with a glycerol level of 30%. The pH of compositions A. - G. is adjusted with NaOH/ citric acid in the range pH 4.5-pH 7.5.**

| Trade Name | Chemical/ INCI Name | A. % Active | B. % Active | C. % Active | D. % Active | F. % Active | G. % Active |
|---|---|---|---|---|---|---|---|
| Sodium cocoyl isethionate | Sodium cocoyl isethionate | 2.4 | 2.4 | 2.4 | 2.4 | 2.4 | 2.4 |
| Sodium methyl cocoyl taurate | Sodium methyl cocoyl taurate | 4.8 | 4.8 | 4.8 | 4.8 | 4.8 | 4.8 |
| Cocamidopropyl betaine | Cocamidopropyl betaine | 1.8 | 1.8 | 1.8 | 1.8 | 1.8 | 1.8 |
| Glycerol | Glycerol | 30 | 30 | 30 | 30 | 30 | 30 |
| NaOH/Citric acid | NaOH/Citric acid | to pH 4.5 | to pH 5.0 | to pH 5.2 | to pH 5.9 | to pH 6.9 | to pH 7.5 |
| DI-water | Water | to 100% | to 100% | to 100% | to 100% | to 100% | to 100% |

After the equilibration period, all of the compositions remained as clear, isotropic solutions with an NTU value of 10 or less. Thus, compositions within the claimed ratio R range are seen to lead to clear, isotropic solutions throughout the interval pH 4.5 to pH 7.5. This flexibility in composition pH will allow freedom to use any of a variety of preservation systems.

### Example 5

Non-limiting examples of compositions of the present invention are described in Table 6. Formulations A-G were processed using the following general procedure:
Part 1- A premix of oils and fatty acid blends was prepared. Examples of oils include petrolatum, soybean oil, and hydrogenated soybean oil. Examples of fatty acids include lauric acid and stearic acid. Where appropriate, preservatives such as BHT (butylated hydroxytoluene) are added to the blend and the mixture heated to about 70°C and mixed. To this mixture, a thickening copolymer, e.g., acrylates/C₁₀-C₃₀ alkyl acrylate crosspolymer or other acrylate copolymer, can be dispersed using a spatula. The premix is kept at about 75°C until ready to be used in formulation.
Part 2- The formulation was assembled from the premix and remaining ingredients. Starch (e.g., sodium hydroxypropyl starch phosphate) was added to a beaker containing water, while being stirred using an overhead mixer. While this mixture was being heated to about 70°C, about 85% of the total glycerol, and all of the isethionate, taurate, and amphoteric surfactants were added. The mixture was kept at 70°C for an additional 15 minutes to assure homogeneity. To this mixture, the hot premix [70°C] of oils, fatty acids, BHT, and acrylates/C₁₀-C₃₀ alkyl acrylate crosspolymer was added. Mixing was continued for an additional 15 minutes before starting to cool. A premix of the remaining glycerol [15% of the total] containing dispersed guar hydroxypropyltrimonium chloride was added at about 60°C. Once the cooling mixtures reached about 50°C, EDTA (ethylenediamine tetraacetic acid) was added, followed by preservative (e.g., DMDM Hydratoin) and Fragrance at 40°C. Mixing was discontinued at about room temperature and product was discharged.

**Table 6. Formulations A to G.**

| | A | B | C | D | E | F | G |
|---|---|---|---|---|---|---|---|
| | | | % | Active | | | |
| Chemical/ INCI Name | | | | | | | |
| | | | | | | | |
| Sodium hydroxypropyl starch phosphate | 1.5 | 1.5 | 1.5 | 1.5 | 1.5 | 3.6 | |
| Acrylates/C10-30 alkyl acrylate crosspolymer | 0.25 | 0.25 | 0.25 | 0.25 | 0.25 | | |
| Acrylates copolymer | | | | | | | 0.5 |
| | | | | | | | |
| Sodium cocoyl isethionate | 2.4 | 2 | 2 | 2 | 3.33 | 2.4 | 2 |
| Sodium methyl cocoyl taurate | 4.8 | 4 | 6 | 4 | 6.66 | 4.8 | 6 |
| Sodium lauroamphoacetate | | 3 | | | | | |
| Cocamidopropyl betaine | 1.8 | | 4 | 3 | 5 | 1.8 | 4 |
| | | | | | | | |
| Glycine soja oil | 1.2 | 1.2 | 1.2 | 1.2 | 1.2 | 3 | |
| Hydrogenated soybean oil | 1.8 | 1.8 | 1.8 | 1.8 | 1.8 | 4 | |
| Petrolatum | 8 | 5 | | | 3 | | |
| | | | | | | | |
| Guar hydroxypropyltrimonium chloride | 0.4 | 0.4 | 0.4 | 0.4 | 0.4 | 0.2 | |
| | | | | | | | |
| Glycerol | 30 | 35 | 30 | 40 | 30 | 30 | 35 |
| | | | | | | | |
| Methylisothiazoline | | | | | | 0.01 | |
| BHT | 0.1 | 0.1 | 0.1 | | | 0.1 | |
| lodopropynyl Butylcarbamate and DMDM Hydantoin | 0.25 | 0.25 | 0.25 | | | | 0.25 |
| Tetrasodium EDTA | 0.05 | 0.05 | 0.05 | 0.05 | 0.05 | 0.05 | 0.05 |
| | | | | | | | |
| Sodium benzoate | | | | 1 | 1 | | |
| | | | | | | | |
| Citric acid monohydrate /NaOH | to pH 7 | to pH 7 | to pH 7 | to pH 5 | to pH 5 | to pH 6 | to pH 7 |
| | | | | | | | |
| NaCl | | | | | | 0.5 | 0.5 |
| | | | | | | | |
| Fragrance | 1.0-1.5 | 1.0-1.5 | 1.0-1.5 | 1.0-1.5 | 1.0-1.5 | 1.0-1.5 | 1.0-1.5 |
| | | | | | | | |
| DI-water | to 100% | to 100% | to 100% | to 100% | to 100% | to 100% | to 100% |

Formulation A illustrates 9% total surfactant with R = 0.5 at pH 7. Formulation B illustrates substitution of amphoacetate for CAPB and Formulation C demonstrates 12% total surfactant with R = 0.333. Formulation D illustrates the reduction of pH to 5.0 and a modified preservation system, while formulaion E is also at pH 5.0 but with 15% surfactant. Formulation F demonstrates a modified preservation system at pH 6. Formulation G is an example with a modified structuring system and no benefiting agent.

### Example 6. Turbidity of Glycerol-free surfactant mixtures.

US 2009/0062406 A1 to Loeffler discloses that mixtures of sodium cocoyl isethionate and sodium methyl cocoyl taurate with alkyl betaines such as coco betaine give clear, isotropic, low viscosity solutions characterized by an NTU reading of 10 or below. The compositions explicitly taught are 20% coco betaine, 5% isethionate, 5% taurate; and 10% coco betaine, 10% isethionate, 10% taurate. Thus all examples have a 1:1 (R = 1.0) isethionate/taurate ratio.

Applicants sought to show how unexpected it would be to find a critical window where ratio of isethionate to taurate provides advantages we have detected. For this example, a series of varying isethionate/taurate contents, 7.5/2.5, 5.0/5.0, 2.5/7.5, and 0/10.0 (R = 3, 1, 0.333, and 0, respectively) were prepared - all with 10% total of the two surfactants. Cocamidopropyl betaine (CAPB) was added at varying levels to each series and the pH of the mixtures adjusted to 7.0. Each individual mixture was heated to 65°C with stirring and then equilibrated at 23°C for two months, by which time no further changes in physical appearance were noted. After equilibration, the transparency of the individual mixtures was measured as described above and reported in the table below.

**Table 7. NTU values of isethionate/taurate mixtures with CAPB.**

| | | ***Isethionate*/*Taurate*** | ***(wt.%*/*wt.%)*** | |
|---|---|---|---|---|
| | *7.5*/*2.5* | *5.0*/*5.0* | *2.5*/*7.5* | *0*/*10.0* |
| *R* | *3* | *1* | *0.333* | *0* |

| ***%CAPB*** | **NTU value** | | | |
|---|---|---|---|---|
| *5* | 20,000 NTU | 17.9 NTU | 13.5 NTU | 3.95 NTU |
| *4* | 40,000 | 18 | 15.9 | 5.5 |
| *3* | 90,000 | 43 | 19 | 5.7 |
| *2* | | 43,000 | 21.6 | 4.5 |
| *1.5* | | | 14.4 | 5.6 |
| *1.2* | | | 34.2 | 6.5 |
| *1* | | | 32 | 9.83 |
| *0.5* | | | 40 | 980 |

The following trends can be seen in Table 7: At a fixed isethionate/taurate ratio, the turbidity increases as the CAPB level is decreased, as indicated by an increasing NTU level. Also, at any fixed CAPB level, the turbidity decreases as the isethionate/taurate ratio is decreased. However, our examples demonstrate that, in glycerol free systems, this trend extends even to taurate only systems (0/10 ratio).

By contrast, and as applicants have shown in Example 1, when high levels of glycerol are desired (as in our invention), applicants have found that isethionate/taurate ratio must be maintained in the claimed range of 1/1.5 to 1/6 (R-0.666 to 0.166) in order to both minimize the need for CAPB (desirable for deposition) and to simultaneously maintain low turbidity. At 0/10 ratio, 40% CAPB is needed to yield isotropic solution with 30% glycerol.

Unexpectedly, at ratio of 1/6, we can have 30% glycerol levels using only 30% CAPB (10% less). At a ratio of 1/5 isethionate to taurate, we can get 35% glycerol (always while remaining isotropic) again using only 30% CAPB. Nothing in the art teaches or suggests such critical windows for enhancing levels of glycerol (while maintaining isotropic composition) and reduced amphoteric levels.

## Claims

1. A composition comprising:
1) 5 to 20% by wt. of a surfactant system comprising:
a) alkali metal alkyl isethionate;
b) alkali metal alkyl taurate; and
c) amphoteric, preferably alkyl betaine,
wherein weight ratio of (a) to (b) Is 1:1.5 to 1:6 (R=0.667 to 0.166),
wherein ratio of (a) and (b)/(c) is 1:1 to 9:1; and
2) 20 to 60% by wt. glycerol.

2. A composition according to claim 1, wherein said composition is clear, wherein clarity is defined by a turbidity of less than 20 NTU, more preferably less than 10 NTU.

3. A composition according to claims 1 or 2, wherein an acyl ratio of (a) to (b) is 1:1.5 to 1:5 (R=0.667 to 0.2).

4. A composition according to any of claims 1 to 3, having a pH of 4.5 to 7.5.

## Patentansprüche

1. Zusammensetzung, umfassend:
1) 5 bis 20 Gew.-% eines Tensidsystems, umfassend:
a) Alkalimetallalkylisethionat;
b) Alkalimetallalkyltaurat; und
c) amphoteres Tensid, bevorzugt Alkylbetain,
wobei das Gewichtsverhältnis von (a) zu (b) 1:1,5 bis 1:6 beträgt (R = 0,667 bis 0,166),
wobei das Verhältnis von (a) und (b) / (c) 1:1 bis 9:1 beträgt; und
2) 20 bis 60 Gew.-% Glycerin.

2. Zusammensetzung nach Anspruch 1, wobei die Zusammensetzung klar ist, wobei die Klarheit durch eine Trübung von weniger als 20 NTU, bevorzugter weniger als 10 NTU, definiert ist.

3. Zusammensetzung nach Anspruch 1 oder 2, wobei ein Acylverhältnis von (a) zu (b) 1:1,5 bis 1:5 beträgt (R = 0,667 bis 0,2).

4. Zusammensetzung nach irgendeinem der Ansprüche 1 bis 3 mit einem pH von 4,5 bis 7,5.

## Revendications

1. Composition comprenant :
1) 5 à 20 % en masse d'un système de tensioactif comprenant :
a) un alkyliséthionate de métal alcalin ;
b) un alkyltaurate de métal alcalin ; et
c) un amphotère, de préférence une alkylbétaïne,
dans laquelle le rapport de masse de (a) à (b) est de 1:1,5 à 1:6 (R=0,667 à 0,166),
dans laquelle le rapport de (a) et (b)/(c) est de 1:1 à 9:1 ; et
2) de 20 à 60 % en masse de glycérol.

2. Composition selon la revendication 1, dans laquelle ladite composition est transparente, dans laquelle la transparence est définie par une turbidité inférieure à 20 NTU, encore mieux inférieure à 10 NTU.

3. Composition selon la revendication 1 ou 2, dans laquelle un taux acyle de (a) à (b) est de 1:1,5 à 1:5 (R=0,667 à 0,2).

4. Composition selon l'une quelconque des revendications 1 à 3, ayant un pH de 4,5 à 7,5.
